# FASCICULE DE BREVET EUROPEEN

(11) **EP 3 642 453 B1**
(45) Date de publication et mention de la délivrance du brevet: **14.09.2022**
(21) Numéro de dépôt: 18725805.8
(22) Date de dépôt: 15.05.2018
(51) Int. Cl.: E21B 49/08, G01N 33/28

(54) **INSTALLATION MOBILE D'ANALYSE D'UN FLUIDE**
MOBILE ANLAGE ZUR ANALYSE EINER FLÜSSIGKEIT
MOBILE FACILITY FOR ANALYSING A FLUID

(30) Priorité: 21.06.2017 FR 1755645
(43) Date de publication de la demande: 29.04.2020
(73) Titulaire: IFP Energies nouvelles, 92852 Rueil-Malmaison (FR); SEMM Logging, 18360 Vesdun (FR)
(72) Inventeur: GARCIA, Bruno, 92200 Neuilly sur Seine (FR); BESSON, Laurent, 03100 Montlucon (FR)
(74) Mandataire: IFP Energies nouvelles
(86) Numéro de dépôt international: PCT/EP2018/062591
(87) Numéro de publication internationale: WO 2018/233937

(56) Documents cités:
- FR-A1- 2 999 224
- US-A1- 2010 132 449
- US-A1- 2012 127 466
- US-B1- 7 210 342

## Description

L'invention concerne le domaine technique de l'exploitation de milieu souterrain, telle l'exploitation de réservoirs souterrains (stockage/soutirage de gaz, exploitation d'hydrocarbures, géothermie...) et la surveillance de ces opérations (par exemple contamination des opérations sur les aquifères). L'invention concerne notamment le domaine de la surveillance de site géologique de stockage de gaz, tel que le dioxyde de carbone (CO₂) ou le méthane. D'autres domaines tels que la récupération assistée d'hydrocarbures par exemple ou l'exploitation de gaz de roches mères sont également concernés par cette invention.

En particulier, l'invention concerne une installation pour analyser un situ un fluide prélevé dans une formation souterraine, selon la revendication 1.

Des fluides présents dans des puits ont besoin d'être prélevés pour déterminer leur composition, afin de caractériser les réservoirs géologiques atteints par le puits et leur évolution dans le temps au cours du procédé industriel de stockage et/ou de production. C'est notamment le cas pour la surveillance de site géologique de stockage de gaz.

Pour suivre l'évolution des fluides injectés au sein d'un milieu poreux, de nombreuses techniques ont été développées par les industriels.

On connaît par exemple des méthodes de surveillance géochimique de sites de stockage géologique de CO₂, basées sur l'étude des espèces volatiles. Des exemples de ces méthodes sont décrits dans les demandes de brevet FR 2.972.758 et FR 2.974.358.

Ces méthodes s'appliquent principalement au niveau de deux compartiments :
- au niveau du réservoir/aquifères salins où l'objectif principal est de quantifier le CO₂ dissous et précipité et donc d'établir un réel bilan de masse ;
- au niveau des aquifères sus-jacents à la roche de couverture ("cap-rock") où l'objectif principal est de diagnostiquer une fuite le plus précocement possible.

Pour mettre en œuvre ces méthodes, il est donc nécessaire de disposer d'un dispositif de prélèvement de fluides sous pression dans un puits foré à travers une formation géologique. Un tel dispositif est appelé échantillonneur ou préleveur.

On connaît des échantillonneurs dits FTS (de l'anglais Flow Through Sampler), permettant d'obtenir des échantillons de fluide à partir d'un puits foré à travers une formation géologique. Un tel dispositif se compose d'une chambre à échantillon avec une soupape à ressort à chaque extrémité. Un mécanisme de verrouillage relie les vannes et les maintient ensemble ouvertes. Au-dessus de la chambre, il existe une horloge pour programmer l'heure de fermeture, et un mécanisme de déclenchement pour libérer les vannes. L'extrémité inférieure a des moyens pour permettre au fluide de pénétrer. Au sommet, il y a une prise de câble pour fixer un câble.

On connaît également des demandes de brevet français FR 299224 et FR 3011029 des dispositifs de prélèvement comportant d'une part un piston contrôlé par un ressort baignant dans une chambre d'huile pour échantillonner le fluide, et d'autre part, un second piston pour expulser le fluide lors du transfert. Le dispositif est maintenu en position ouverte ou fermée par le ressort comprimé logé dans la chambre remplie d'huile. L'huile contenue dans la chambre du ressort permet d'amortir l'effet de décompression et de réaliser le prélèvement sans à-coup. Le dispositif permet la récupération du fluide échantillonné grâce à l'action mécanique d'un piston solide au travers d'une vanne manuelle. Ce dispositif présente également l'avantage de pouvoir être descendu en position ouverte dans le milieu souterrain, de manière à s'affranchir des problèmes d'ouverture et pour permettre un remplissage complet de la chambre d'échantillonnage.

Pour mettre en œuvre ces méthodes de surveillance, il est ensuite nécessaire d'analyser le fluide récupéré. Actuellement, le fluide prélevé au moyen de ces préleveurs est analysé dans un laboratoire d'analyse souvent éloigné du site de prélèvement. Cet éloignement génère de nombreux inconvénients :
- en premier lieu le transport de l'échantillon prélevé qui risque de détériorer l'échantillon,
- la nécessité de réceptionner le préleveur, qui est un équipement considéré comme dangereux car il s'agit d'un équipement sous pression,
- des problèmes de douanes, de législation lorsque le laboratoire d'analyse est situé dans un pays différent de celui du site de prélèvement,
- un risque de contamination du fluide prélevé par une saumure synthétique utilisée pour pousser le fluide en place dans le préleveur, et
- un temps de transport important, qui empêche une analyse en temps réel, ce qui ne permet pas un contrôle du site souterrain en temps réel, etc.

On connaît également de la demande de brevet US 2012/0127466 un système d'analyse PVT, comprenant une chambre de contrôle environnemental portable, un premier récipient sous pression disposé à l'intérieur de la chambre de contrôle environnemental portable, un deuxième récipient sous pression disposé à l'intérieur de la chambre de contrôle environnemental portable, le deuxième récipient sous pression étant en communication hydraulique avec le premier récipient sous pression, un viscosimètre configuré pour mesurer la viscosité du fluide s'écoulant entre le premier récipient sous pression et le deuxième récipient sous pression, et un système optique configuré pour mesurer les propriétés optiques du fluide s'écoulant entre le premier récipient sous pression et le second récipient sous pression.

Pour pallier ces inconvénients, l'invention concerne une installation mobile d'analyse d'un fluide. L'installation est mobile afin d'être apte à être déplacée sur le site du prélèvement, afin de ne pas avoir à transporter le préleveur pour l'analyse. Pour réaliser l'analyse, l'installation comporte notamment au moins deux cellules à piston, des moyens d'analyse PVT et des moyens d'analyse géochimique. Ainsi, l'installation permet une analyse PVT et une analyse géochimique du fluide à analyser.

### Le système selon l'invention

L'invention concerne une installation mobile d'analyse d'un fluide. Ladite installation mobile d'analyse comporte au moins :
- un premier circuit comprenant de l'huile,
- au moins deux cellules à piston, chaque cellule à piston comprenant un piston délimitant deux volumes, un premier volume étant relié audit premier circuit,
- des moyens de connexion à un contenant comprenant ledit fluide à analyser pour transférer ledit fluide à analyser dans les deuxièmes volumes desdites cellules à piston,
- un deuxième circuit comprenant une saumure ou de l'eau, notamment de l'eau pure, ledit deuxième circuit étant relié au contenant pour contrôler l'injection dudit fluide à analyser,
- des moyens d'analyse PVT pour déterminer le ratio gaz - eau et/ou gaz - huile dudit fluide à analyser contenu dans une première cellule à piston,
- des moyens d'analyse géochimique pour déterminer au moins une partie de la composition dudit fluide à analyser contenu dans une deuxième cellule à piston.

Selon un mode de réalisation, lesdits moyens d'analyse géochimique comprennent des moyens de dégazage dudit fluide contenu dans ledit deuxième volume de ladite deuxième cellule à piston.

Conformément à une mise en œuvre, lesdits premier et deuxième circuits comportent des pompes autorégulées.

Selon un aspect, ladite installation mobile d'analyse comporte trois cellules à piston, deux desdites cellules à piston étant reliées à des moyens d'analyse géochimique.

Avantageusement, lesdits moyens d'analyse PVT comportent des moyens de mesure de la pression de saturation et du volume des gaz contenus dans ledit fluide à analyser.

Selon une caractéristique, l'installation mobile d'analyse comporte un circuit d'ajout d'un gaz inerte, de préférence de l'azote ou de l'argon, dans lesdits moyens d'analyse géochimique.

Conformément à un mode de réalisation, lesdits moyens d'analyse géochimique comportent des moyens d'analyse de la composition des gaz contenus dans ledit fluide à analyser, lesdits moyens étant aptes à déterminer la quantité desdits gaz choisis parmi les gaz suivants CO₂, N₂, O₂, les hydrocarbures ayant des chaînes C₁ à C₄, He, Ne, Ar, Kr, Xe et/ou à déterminer la signature isotopique *δ*¹³*C*.

Selon une mise en œuvre, lesdits moyens d'analyse géochimique comportent un piège à eau et un cylindre d'échantillonnage.

De manière avantageuse, ladite installation mobile d'analyse est contenue dans un caisson sensiblement parallélépipédique dont la longueur est inférieure ou égale à 20 m, la hauteur est inférieure ou égale à 3 m, et la largeur est inférieure ou égale à 5 m.

De préférence, tous les équipements de ladite installation mobile d'analyse sont fixes par rapport audit caisson.

Selon un aspect, ladite installation mobile d'analyse comporte un premier ensemble de vannes pour transférer ledit fluide à analyser vers lesdites cellules à piston.

Selon une caractéristique, ladite installation mobile d'analyse comporte un deuxième ensemble de vannes pour transférer ledit fluide à analyser depuis lesdites cellules à piston vers lesdites moyens d'analyse PVT et lesdits moyens d'analyse géochimique.

Selon un mode de réalisation, ledit contenant dudit fluide à analyser est un préleveur de fond.

En outre, l'invention concerne une utilisation d'une installation mobile d'analyse selon l'une des caractéristiques précédentes, pour analyser un fluide prélevé dans une formation souterraine d'un site de stockage de gaz, de production d'hydrocarbures, de géothermie, de production d'hydrogène naturel, selon la revendication 14.

Avantageusement, on analyse la pression de saturation, le volume des gaz présents dans le fluide à prélever, et la composition des gaz présents dans ledit fluide prélevé.

Selon un mode de réalisation, on déplace ladite installation mobile d'analyse sur un site de prélèvement dudit fluide à analyser.

Conformément à une mise en œuvre, on met en œuvre les étapes suivantes :
a) on installe un contenant dudit fluide à analyser dans ladite installation mobile d'analyse ;
b) on transfère ledit fluide à analyser dudit contenant vers lesdites cellules à piston ;
c) on réalise une analyse PVT à partir dudit fluide contenu dans une première cellule à piston ; et
d) on réalise au moins une analyse géochimique à partir dudit fluide contenu dans au moins une deuxième cellule à piston.

### Présentation succincte des figures

D'autres caractéristiques et avantages de l'installation selon l'invention, apparaîtront à la lecture de la description ci-après d'exemples non limitatifs de réalisations, en se référant à la figure annexée et décrite ci-après.

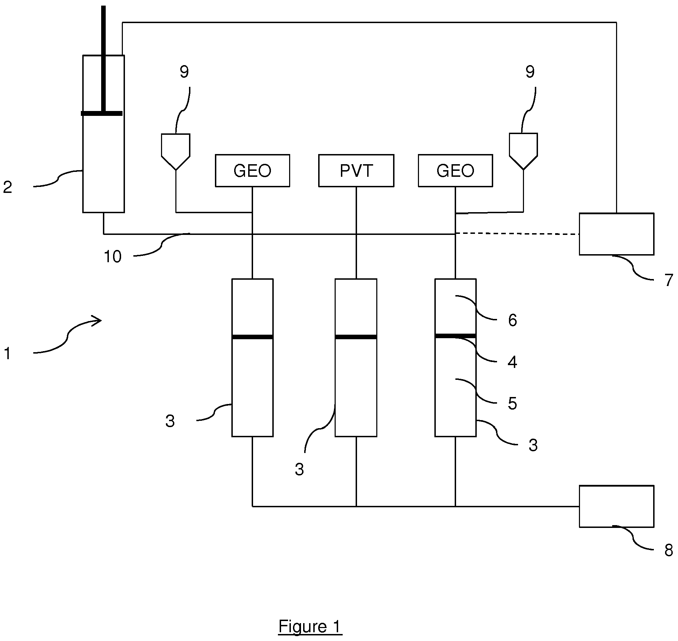

La figure 1 illustre une installation d'analyse d'un fluide selon un mode de réalisation de l'invention.

### Description détaillée de l'invention

La présente invention concerne une installation mobile d'analyse d'un fluide, notamment les gaz et les gaz dissous dans un fluide. L'installation est dite mobile, car elle est apte à être déplacée d'un site à un autre, sans démontage des équipements. Ainsi, il est possible de déplacer l'installation sur un site dans lequel on prélève un fluide dans le but de réaliser des analyses in situ du fluide prélevé. De cette manière, l'invention présente notamment les avantages suivants :
- l'installation mobile d'analyse ne nécessite pas de transport et de réception de l'échantillon prélevé,
- l'installation mobile d'analyse permet d'éviter des problèmes de douanes, de législation, et
- l'installation mobile d'analyse limite les risques de contamination du fluide prélevé,
- l'installation mobile d'analyse permet une analyse quasiment en temps réel, ce qui permet un contrôle du site souterrain quasiment en temps réel.

Selon l'invention, l'installation mobile comporte au moins les équipements suivants :
- un premier circuit comprenant de l'huile,
- un deuxième circuit comprenant une saumure ou de l'eau, en particulier de l'eau pure,
- au moins deux cellules à piston, chaque cellule comprenant un piston délimitant deux volumes : un premier volume est relié au premier circuit comprenant de l'huile, et le deuxième volume est relié à un contenant comprenant le fluide à analyser,
- des moyens de connexion au contenant comprenant le fluide à analyser pour transférer le fluide à analyser dans une des cellules à piston, en particulier dans les deuxièmes volumes des cellules à piston,
- des moyens d'analyse PVT (pression, volume, température) pour déterminer le ratio gaz - eau et/ou gaz huile du fluide à analyser, les moyens d'analyse PVT sont reliés à un deuxième volume d'une première cellule à piston, et
- des moyens d'analyse géochimique pour déterminer au moins partiellement la composition du fluide à analyser contenu dans un deuxième volume d'au moins une des cellules à piston.

Ainsi, pour réaliser les analyses du fluide à analyser, le fluide est transféré depuis son contenant dans les cellules à piston, les cellules à piston étant préalablement vides, puis des cellules à piston dans des moyens d'analyse (PVT et géochimique). Les cellules à piston permettent d'homogénéiser le volume de ciel obtenu en cas de faible quantité de gaz présents dans le fluide à analyser (par réglage de la pression au sein des cellules à piston). De plus, les cellules à piston permettent d'adapter les pressions d'injection pour les analyses (PVT et géochimique), en adaptant les pressions des fluides des premier et deuxième circuits.

L'invention permet de réaliser in situ différentes analyses du fluide à analyser.

Le deuxième circuit est relié au contenant de manière à contrôler l'injection du fluide à analyser dans les cellules de mesure. De manière optionnelle, le deuxième circuit peut être relié aux moyens de connexion pour permettre de purger le système.

Selon un aspect de la divulgation, les cellules à piston peuvent avoir un volume qui est compris entre un sixième et un tiers du volume du contenant du fluide à analyser. Selon un exemple non limitatif, le contenant du fluide à analyser peut avoir un volume de sensiblement 600 cm³, et l'installation peut comprendre trois cellules à piston ayant un volume respectif de sensiblement 150 cm³.

De préférence, le fluide à analyser peut être un fluide prélevé dans une formation souterraine, en particulier sur un site de stockage de gaz, sur un site de production d'hydrocarbures (conventionnels ou non conventionnels, au moyen d'un procédé de récupération assistée des hydrocarbures EOR ou non), sur un site de géothermie, sur un site de production d'hydrogène naturel, ou tout site analogue. Le fluide prélevé d'une formation souterraine et à analyser peut être sous la forme d'une saumure, c'est-à-dire une solution aqueuse comprenant une forte concentration de sels. De plus, le fluide prélevé peut comporter des hydrocarbures. Alternativement, le fluide à analyser peut être de tout autre nature.

De manière avantageuse le contenant du fluide à analyser peut être un préleveur de fond. Ainsi, il est possible de connecter directement le fluide prélevé à analyser à l'installation d'analyse sans transport du contenant et sans transfert du fluide du préleveur de fond à un contenant intermédiaire. Le préleveur de fond peut être notamment conforme à l'un des préleveurs de fond décrits dans les demandes de brevet FR 2999224 et FR 3011029. Alternativement, le contenant peut être de tout autre nature.

Avantageusement, pour réaliser les mesures géochimiques, les moyens d'analyse géochimique peuvent comprendre des moyens de dégazage du fluide contenu dans le deuxième volume d'une cellule à piston. Ces moyens de dégazage peuvent consister à des moyens de contrôle de la pression dans la cellule à piston. En effet, en diminuant la pression au sein de la cellule à piston, le fluide à étudier est dégazé.

De plus, afin de pouvoir faciliter la mesure, si le volume de gaz présents dans le fluide est faible, l'installation peut comprendre un circuit d'ajout d'un gaz inerte, en amont des moyens d'analyse géochimiques. Le gaz inerte ajouté peut être un gaz inerte ou un gaz rare, tel que l'azote ou l'argon.

Conformément à une mise en œuvre, les moyens d'analyse géochimique peuvent comporter des moyens d'analyse de la composition des gaz présents dans le fluide à analyser, ces moyens étant aptes à déterminer la quantité desdits gaz choisis parmi les gaz suivants CO₂, N₂, O₂, les hydrocarbures gazeux ayant des chaînes C₁ à C₄, He, Ne, Ar, Kr, Xe et/ou à déterminer la signature isotopique *δ*¹³*C*. Ainsi, il est possible de déterminer la composition des gaz généralement présents dans le fluide à analyser, en particulier lorsqu'il s'agit d'un fluide prélevé dans une formation souterraine.

Selon un mode de réalisation, les moyens d'analyse géochimique peuvent comporter un piège à eau, permettant d'isoler le gaz à analyser. De plus, les moyens d'analyse géochimique peuvent comporter un cylindre d'échantillonnage pour réaliser les mesures quantitatives correspondantes. Les mesures peuvent réalisées par un analyseur classique, qui peut être connecté et intégré à l'installation.

Avantageusement, les moyens d'analyse PVT peuvent comporter des moyens de mesure de la pression de saturation des gaz présents dans le fluide à analyser, et du volume des gaz présents dans le fluide à analyser. Au moyen des mesures de volume, on peut déterminer le ratio gaz - eau et/ou le ratio gaz - huile du fluide à analyser. Au moyen de la pression de saturation, on peut connaître le point de bulle des gaz présents dans le fluide à analyser.

Selon un mode de réalisation, on réalise l'analyse PVT au préalable des analyses géochimiques. De cette manière, on peut adapter le volume de fluide nécessaire pour les analyses géochimiques aussi bien en terme qualitatif qu'en terme quantitatif.

Afin d'automatiser les analyses, les premier et deuxième circuits peuvent comprendre des pompes autorégulées.

De plus, pour contrôler les transferts du fluide entre le contenant et les cellules à piston, l'installation peut comprendre un premier ensemble de vannes. Ces vannes sont commandés afin de réaliser les étapes permettant les différentes mesures.

En outre, pour contrôler les transferts du fluide entre les cellules à piston et les moyens d'analyse PVT et les moyens d'analyse géochimique, l'installation peut comprendre un deuxième ensemble de vannes. Ces vannes sont commandés afin de réaliser les étapes permettant les différentes mesures.

Selon une mise en œuvre de l'invention, l'installation peut comporter trois cellules à piston. Pour cette mise en œuvre, une cellule à piston peut être reliée à des moyens d'analyse PVT, et deux cellules à piston peuvent être reliées à des moyens d'analyse géochimiques distinctes. Cette conception permet de doubler l'échantillonnage.

En variante, l'installation mobile d'analyse peut comporter entre 2 et 6 cellules à piston.

Afin de pouvoir être facilement transportable, l'installation mobile peut être contenue dans un caisson sensiblement parallélépipédique. Par exemple, l'installation mobile d'analyse peut être contenue dans une cabine de chantier, ou dans un conteneur utilisé notamment pour le fret maritime. Le caisson peut avoir une longueur inférieure ou égale à 8 m, une hauteur inférieure ou égale à 2 m et une largeur inférieure ou égale à 3 m.

Pour cette mise en œuvre, les équipements de l'installation mobile d'analyse peuvent être fixes par rapport au caisson.

La figure 1 illustre, schématiquement et de manière non limitative, une installation mobile d'analyse selon un mode de réalisation de l'invention. Sur cette figure, les moyens de contrôle et les vannes autorisant le transfert ne sont pas représentés. L'installation mobile d'analyse 1 comporte un contenant 2 dans lequel est contenu le fluide à analyser. Le contenant 2 comprend des moyens de transfert du fluide : un piston selon l'exemple illustré. Le contenant 2 peut être un préleveur de fond. L'installation mobile d'analyse 1 illustré comporte trois cellules à piston 3. Chaque cellule à piston 3 comprend un piston 4 qui délimite deux volumes 5 et 6. Le premier volume 5 est relié à un premier circuit 8 comprenant de l'huile. Le deuxième volume 6 est relié au contenant 2 par des moyens de connexion 10. De plus l'installation comprend un deuxième circuit 7 comprenant une saumure ou de l'eau, par exemple de l'eau pure. Le deuxième circuit 7 est relié au contenant 2 de manière à contrôler l'injection du fluide à analyser dans les cellules de mesure. De manière optionnelle, le deuxième circuit 7 peut être relié aux moyens de connexion 10 pour permettre de purger le système. Les premier 8 et deuxième 7 circuits comprennent des pompes autorégulées (non représentées). La cellule à piston 3 centrale est reliée à des moyens d'analyse PVT pour déterminer le ratio gaz - eau et/ou le ratio gaz - huile du fluide à analyser. Les deux autres cellules à piston 3 sont reliées à des moyens d'analyse géochimique GEO pour analyser au moins partiellement la composition du fluide à analyser. Selon le mode de réalisation illustré, l'installation mobile 1 comporte, de manière optionnelle, des moyens d'ajout d'un gaz inerte 9 en amont des moyens d'analyse géochimique. Par exemple, le gaz inerte peut être de l'azote ou de l'argon.

En outre, la divulgation concerne l'utilisation d'une installation mobile d'analyse, selon l'une quelconque des combinaisons des variantes décrites précédemment, pour analyser un fluide prélevé dans une formation souterraine d'un site de stockage de gaz (par exemple du CO₂), d'un site de production des hydrocarbures (conventionnels ou non, au moyen d'une récupération assistée des hydrocarbures EOR ou non), d'un site de géothermie, d'un site de production d'hydrogène naturel, ou tout autre site analogue. Il peut s'agir notamment de l'installation mobile d'analyse schématisée en figure 1.

L'utilisation de l'installation mobile peut être mise en œuvre dans le cadre de la surveillance d'un de ces sites, et peut permettre notamment de détecter une fuite d'un fluide dans une formation souterraine. A la suite de la détection d'une fuite, une action de remédiation peut être rapidement mise en œuvre pour arrêter la fuite, grâce aux analyses réalisées in situ. Par exemple dans le cas du stockage de gaz dans la formation souterraine, on peut détecter une fuite de gaz stocké, et il est alors possible de limiter la quantité de gaz stockée pour empêcher une fuite plus importante.

Au moyen de l'installation mobile, on peut analyser la pression de saturation, les ratios gaz - eau et gaz - huile des gaz présents dans le fluide prélevé (par les moyens d'analyse PVT). On peut aussi analyser la composition du fluide prélevé (par les moyens d'analyse géochimique).

L'installation étant mobile, l'utilisation de cette installation mobile d'analyse peut comprendre une étape dans laquelle on déplace l'installation mobile d'analyse directement sur le site (à proximité du site) de prélèvement du fluide.

Selon un mode de réalisation, l'utilisation de l'installation mobile d'analyse peut être réalisée en mettant en œuvre les étapes suivantes :
a) on installe un contenant du fluide à analyser dans ladite installation mobile d'analyse ;
b) on transfère le fluide à analyser du contenant vers les cellules à piston ;
c) on réalise une analyse PVT à partir du fluide contenu dans une première cellule à piston pour déterminer le ratio gaz - eau et/ou gaz - huile du fluide à analyser ; et
d) on réalise au moins une analyse géochimique à partir du fluide contenu dans au moins une deuxième cellule à piston pour déterminer au moins partiellement la composition du fluide à analyser.

L'utilisation de l'installation mobile d'analyse peut comprendre une étape préalable d'un prélèvement d'un fluide à analyser dans une formation souterraine.

Lors de l'étape a), le contenant peut être un préleveur de fond qui a prélevé un fluide dans une formation souterraine.

Lors de l'étape c), on mesure par l'analyse PVT la pression de saturation et le volume des gaz présents dans le fluide à analyser.

Selon un mode de réalisation, on réalise l'analyse PVT au préalable des analyses géochimiques, et on adapte le volume de fluide nécessaire pour les analyses géochimiques aussi bien en terme qualitatif qu'en terme quantitatif.

Lors de l'étape d), on détermine par l'analyse géochimique la quantité desdits gaz choisis parmi les gaz suivants CO₂, N₂, O₂, les hydrocarbures gazeux ayant des chaînes C₁ à C₄, He, Ne, Ar, Kr, Xe et/ou on détermine la signature isotopique *δ*¹³*C*. Ainsi, il est possible de déterminer la composition des gaz généralement présents dans le fluide à analyser, en particulier lorsqu'il s'agit d'un fluide prélevé dans une formation souterraine.

Conformément un mode de réalisation, l'utilisation peut comprendre une étape d'ajout d'un gaz inerte, tel l'azote ou l'argon, dans le fluide à analyser avant l'étape d) d'analyse géochimique. Cet ajout permet de faciliter la mesure quand le volume de gaz à analyser est faible.

L'utilisation peut comporter également une étape de préparation de l'installation. Lors de cette étape, on peut purger les cellules à piston pour faire sortir les éventuels fluides présents initialement, par déplacement du piston des cellules à piston au moyen notamment de l'huile du premier circuit. Ensuite, on peut saturer les cellules à piston de saumure au moyen du deuxième circuit. Enfin, on régule la pression au sein des cellules à piston, en ajustant les pressions des premier et deuxième circuits.

## Revendications

1. Installation mobile d'analyse d'un fluide apte à être déplacée d'un site à un autre, sans démontage des équipements, ladite installation mobile d'analyse (1) comportant au moins:
- un premier circuit comprenant de l'huile (8),
- au moins deux cellules à piston (3), chaque cellule à piston (3) comprenant un piston (4) délimitant deux volumes (5, 6), un premier volume (5) étant relié audit premier circuit (8), et un deuxième volume relié à un contenant (2) comprenant ledit fluide à analyser,
- des moyens de connexion (10) audit contenant (2) comprenant ledit fluide à analyser pour transférer ledit fluide à analyser dans les deuxièmes volumes (6) desdites cellules à piston (3),
- un deuxième circuit comprenant une saumure (7) ou de l'eau, notamment de l'eau pure, ledit deuxième circuit étant relié au contenant (2) pour contrôler l'injection dudit fluide à analyser depuis ledit contenant (2) dans lesdites au moins deux cellules à piston (3),
- des moyens d'analyse PVT (PVT) pour déterminer le ratio gaz - eau et/ou gaz - huile dudit fluide à analyser contenu dans une première cellule à piston (3), les moyens d'analyse PVT sont reliés à un deuxième volume de ladite première cellule à piston (3),
- des moyens d'analyse géochimique (GEO) pour déterminer au moins une partie de la composition dudit fluide à analyser contenu dans un deuxième volume dans une deuxième cellule à piston (3).

2. Installation selon la revendication 1, dans laquelle lesdits moyens d'analyse géochimique (GEO) comprennent des moyens de dégazage dudit fluide contenu dans ledit deuxième volume de ladite deuxième cellule à piston.

3. Installation selon l'une des revendications précédentes, dans laquelle lesdits premier et deuxième circuits (7, 8) comportent des pompes autorégulées.

4. Installation selon l'une des revendications précédentes, dans laquelle ladite installation mobile d'analyse (1) comporte trois cellules à piston (3), deux desdites cellules à piston (3) étant reliées à des moyens d'analyse géochimique (GEO).

5. Installation selon l'une des revendications précédentes, dans laquelle lesdits moyens d'analyse PVT comportent des moyens de mesure de la pression de saturation et du volume des gaz contenus dans ledit fluide à analyser.

6. Installation selon l'une des revendications précédentes, dans laquelle l'installation mobile d'analyse (1) comporte un circuit d'ajout d'un gaz inerte, de préférence de l'azote ou de l'argon, dans lesdits moyens d'analyse géochimique (GEO).

7. Installation selon l'une des revendications précédentes, dans laquelle lesdits moyens d'analyse géochimique (GEO) comportent des moyens d'analyse de la composition des gaz contenus dans ledit fluide à analyser, lesdits moyens étant aptes à déterminer la quantité desdits gaz choisis parmi les gaz suivants CO₂, N₂, O₂, les hydrocarbures ayant des chaînes C₁ à C₄, He, Ne, Ar, Kr, Xe et/ou à déterminer la signature isotopique *δ*¹³*C*.

8. Installation selon l'une des revendications précédentes, dans laquelle lesdits moyens d'analyse géochimique (GEO) comportent un piège à eau pour isoler le gaz à analyser et un cylindre d'échantillonnage pour réaliser les mesures quantitatives correspondantes.

9. Installation selon l'une des revendications précédentes, dans laquelle ladite installation mobile d'analyse (1) est contenue dans un caisson sensiblement parallélépipédique dont la longueur est inférieure ou égale à 20 m, la hauteur est inférieure ou égale à 3 m, et la largeur est inférieure ou égale à 5 m.

10. Installation selon la revendication 9, dans laquelle tous les équipements de ladite installation mobile d'analyse (1) sont fixes par rapport audit caisson.

11. Installation selon l'une des revendications précédentes, dans laquelle ladite installation mobile d'analyse (1) comporte un premier ensemble de vannes pour transférer ledit fluide à analyser vers lesdites cellules à piston (3).

12. Installation selon l'une des revendications précédentes, dans laquelle ladite installation mobile d'analyse (1) comporte un deuxième ensemble de vannes pour transférer ledit fluide à analyser depuis lesdites cellules à piston (3) vers lesdites moyens d'analyse PVT et lesdits moyens d'analyse géochimique (GEO).

13. Installation selon l'une des revendications précédentes, dans laquelle ledit contenant (2) dudit fluide à analyser est un préleveur de fond.

14. Utilisation d'une installation mobile d'analyse (1) selon l'une des revendications précédentes, pour analyser un fluide prélevé dans une formation souterraine d'un site de stockage de gaz, de production d'hydrocarbures, de géothermie, de production d'hydrogène naturel.

15. Utilisation selon la revendication 14, dans laquelle on analyse la pression de saturation, le volume des gaz présents dans le fluide à prélever, et la composition des gaz présents dans ledit fluide prélevé.

16. Utilisation selon l'une des revendications 14 ou 15, dans laquelle on déplace ladite installation mobile d'analyse (1) sur un site de prélèvement dudit fluide à analyser.

17. Utilisation selon l'une des revendications 14 à 16, dans laquelle on met en œuvre les étapes suivantes :
a) on installe un contenant (2) dudit fluide à analyser dans ladite installation mobile d'analyse (1) ;
b) on transfère ledit fluide à analyser dudit contenant (2) vers lesdites cellules à piston (3) ;
c) on réalise une analyse PVT à partir dudit fluide contenu dans une première cellule à piston (3) ; et
d) on réalise au moins une analyse géochimique (GEO) à partir dudit fluide contenu dans au moins une deuxième cellule à piston (3).

## Patentansprüche

1. Mobile Anlage zur Analyse eines Fluids, die geeignet ist, ohne Demontage der Einrichtungen von einem Ort zu einem anderen verlagert zu werden, wobei die mobile Analyseanlage (1) wenigstens Folgendes aufweist:
- einen ersten Kreis, der Öl umfasst (8),
- wenigstens zwei Kolbenzellen (3), wobei jede Kolbenzelle (3) einen Kolben (4) umfasst, der zwei Volumen (5, 6) begrenzt, wobei ein erstes Volumen (5) mit dem ersten Kreis (8) verbunden ist und ein zweites Volumen mit einem Behälter (2) verbunden ist, der das zu analysierende Fluid umfasst,
- Mittel zur Anschluss (10) an den Behälter (2), der das zu analysierende Fluid umfasst, um das zu analysierende Fluid in die zweiten Volumen (6) der Kolbenzellen (3) zu übertragen,
- einen zweiten Kreis, der eine Sole (7) oder Wasser, insbesondere Reinwasser umfasst, wobei der zweite Kreis mit dem Behälter (2) verbunden ist, um die Einspritzung des zu analysierenden Fluids vom Behälter (2) in die wenigstens zwei Kolbenzellen (3) zu steuern,
- PVT-Analysemittel (PVT) zur Bestimmung des Gas-Wasser- und/oder Gas-Öl-Verhältnisses des zu analysierenden Fluids, das in einer ersten Kolbenzelle (3) enthalten ist, wobei die PVT-Analysemittel mit einem zweiten Volumen der ersten Kolbenzelle (3) verbunden sind,
- geochemische Analysemittel (GEO) zur Bestimmung wenigstens eines Anteils der Zusammensetzung des zu analysierenden Fluids, das in einem zweiten Volumen in einer zweiten Kolbenzelle (3) enthalten ist.

2. Anlage nach Anspruch 1, wobei die geochemischen Analysemittel (GEO) Mittel zur Entgasung des Fluids, das im zweiten Volumen der zweiten Kolbenzelle enthalten ist, umfassen.

3. Anlage nach einem der vorhergehenden Ansprüche, wobei der erste und zweite Kreis (7, 8) selbstregelnde Pumpen aufweisen.

4. Anlage nach einem der vorhergehenden Ansprüche, wobei die mobile Analyseanlage (1) drei Kolbenzellen (3) aufweist, wobei zwei der Kolbenzellen (3) mit geochemischen Analysemitteln (GEO) verbunden sind.

5. Anlage nach einem der vorhergehenden Ansprüche, wobei die PVT-Analysemittel Mittel zur Messung des Sättigungsdruck und des Volumens der Gase, die im zu analysierenden Fluid enthalten sind, aufweisen.

6. Anlage nach einem der vorhergehenden Ansprüche, wobei die mobile Analyseanlage (1) einen Kreis zur Hinzugabe eines Inertgases, vorzugsweise Stickstoff oder Argon, in die geochemischen Analysemittel (GEO) aufweist.

7. Anlage nach einem der vorhergehenden Ansprüche, wobei die geochemischen Analysemittel (GEO) Mittel zur Analyse der Zusammensetzung der Gase, die im zu analysierenden Fluid enthalten sind, aufweisen, wobei die Mittel geeignet sind, die Menge der Gase zu bestimmen, die aus den folgenden Gasen gewählt sind CO₂, N₂, O₂, Kohlenwasserstoffen mit C₁ bis C₄ -Ketten, He, Ne, Ar, Kr, Xe, und/oder die Isotopensignatur δ¹³C zu bestimmen.

8. Anlage nach einem der vorhergehenden Ansprüche, wobei die geochemischen Analysemittel (GEO) eine Wasserfalle zur Isolierung des zu analysierenden Gases und einen Probenentnahmezylinder zur Durchführung der entsprechenden quantitativen Messungen aufweisen.

9. Anlage nach einem der vorhergehenden Ansprüche, wobei die mobile Analyseanlage (1) in einem im Wesentlichen quaderförmigen Kasten enthalten ist, dessen Länge kleiner als oder gleich 20 m ist, dessen Höhe kleiner als oder gleich 3 m ist und dessen Breite kleiner als oder gleich 5 m ist.

10. Anlage nach Anspruch 9, wobei alle Einrichtungen der mobilen Analyseanlage (1) im Verhältnis zum Kasten fest sind.

11. Anlage nach einem der vorhergehenden Ansprüche, wobei die mobile Analyseanlage (1) einen ersten Satz Ventile zur Übertragung des zu analysierenden Fluids zu den Kolbenzellen (3) aufweist.

12. Anlage nach einem der vorhergehenden Ansprüche, wobei die mobile Analyseanlage (1) einen zweiten Satz Ventile zur Übertragung des zu analysierenden Fluids von den Kolbenzellen (3) zu den PVT-Analysemitteln und den geochemischen Analysemitteln (GEO) aufweist.

13. Anlage nach einem der vorhergehenden Ansprüche, wobei der Behälter (2) des zu analysierenden Fluids eine Tiefenentnahmevorrichtung ist.

14. Nutzung einer mobilen Analyseanlage (1) nach einem der vorhergehenden Ansprüche zur Analyse eines Fluids, das in einer unterirdischen Formation einer Stätte zur Speicherung von Gas, zur Produktion von Kohlenwasserstoffen, einer Geothermiestätte, einer Stätte zur Produktion von natürlichem Wasserstoff entnommen wurde.

15. Nutzung nach Anspruch 14, wobei der Sättigungsdruck, das Volumen der Gase, die im zu entnehmenden Fluid vorhanden sind, und die Zusammensetzung der Gase, die im zu entnehmenden Fluid vorhanden sind, analysiert werden.

16. Nutzung nach einem der Ansprüche 14 oder 15, wobei die mobile Analyseanlage (1) an einen Entnahmeort des zu analysierenden Fluids verlagert wird.

17. Nutzung nach einem der Ansprüche 14 bis 16, wobei die folgenden Schritte durchgeführt werden:
a) ein Behälter (2) des zu analysierenden Fluids wird in die mobile Analyseanlage (1) eingesetzt;
b) das zu analysierende Fluid wird vom Behälter (2) zu den Kolbenzellen (3) übertragen;
c) eine PVT-Analyse wird anhand des Fluids durchgeführt, das in einer ersten Kolbenzelle (3) enthalten ist; und
d) wenigstens eine geochemische Analyse (GEO) wird anhand des Fluids durchgeführt, das in wenigstens einer zweiten Kolbenzelle (3) enthalten ist.

## Claims

1. Mobile installation for the analysis of a fluid capable of being moved from one site to another, without dismantling the items of equipment, said mobile analytical installation (1) comprising at least:
- one first circuit comprising oil (8),
- at least two plug-flow cells (3), each plug-flow cell (3) comprising a piston (4) delimiting two volumes (5, 6), a first volume (5) being connected to said first circuit (8) and a second volume connected to a container (2) comprising said fluid to be analysed,
- means for connecting (10) to said container (2) comprising said fluid to be analysed in order to transfer said fluid to be analysed into the second volumes (6) of said plug-flow cells (3),
- a second circuit comprising a brine (7) or water, in particular pure water, said second circuit being connected to the container (2) in order to control the injection of said fluid to be analysed from said container (2) into said at least two plug-flow cells (3) ,
- PVT analytical means (PVT) for determining the gas/water and/or gas/oil ratio of said fluid to be analysed contained in a first plug-flow cell (3); the PVT analytical means are connected to a second volume of said first plug-flow cell (3),
- geochemical analytical means (GEO) for determining at least a part of the composition of said fluid to be analysed contained in a second volume in a second plug-flow cell (3).

2. Installation according to Claim 1, in which said geochemical analytical means (GEO) comprise means for degassing said fluid contained in said second volume of said second plug-flow cell.

3. Installation according to either of the preceding claims, in which said first and second circuits (7, 8) comprise self-regulating pumps.

4. Installation according to one of the preceding claims, in which said mobile analytical installation (1) comprises three plug-flow cells (3), two of said plug-flow cells (3) being connected to geochemical analytical means (GEO).

5. Installation according to one of the preceding claims, in which said PVT analytical means comprise means for measuring the saturation pressure and the volume of the gases contained in said fluid to be analysed.

6. Installation according to one of the preceding claims, in which the mobile analytical installation (1) comprises a circuit for addition of an inert gas, preferably nitrogen or argon, into said geochemical analytical means (GEO).

7. Installation according to one of the preceding claims, in which said geochemical analytical means (GEO) comprise means for analysis of the composition of the gases contained in said fluid to be analysed, said means being capable of determining the amount of said gases chosen from the following gases: CO₂, N₂, O₂, hydrocarbons having C₁ to C₄ chains, He, Ne, Ar, Kr and Xe, and/or of determining the *δ*¹³*C* isotopic signature.

8. Installation according to one of the preceding claims, in which said geochemical analytical means (GEO) comprise a water trap for isolating the gas to be analysed and a sampling cylinder for carrying out the corresponding quantitative measurements.

9. Installation according to one of the preceding claims, in which said mobile analytical installation (1) is contained in a substantially parallelepipedal casing, the length of which is less than or equal to 20 m, the height of which is less than or equal to 3 m and the width of which is less than or equal to 5 m.

10. Installation according to Claim 9, in which all the items of equipment of said mobile analytical installation (1) are fixed with respect to said casing.

11. Installation according to one of the preceding claims, in which said mobile analytical installation (1) comprises a first set of valves for transferring said fluid to be analysed towards said plug-flow cells (3).

12. Installation according to one of the preceding claims, in which said mobile analytical installation (1) comprises a second set of valves for transferring said fluid to be analysed from said plug-flow cells (3) towards said PVT analytical means and said geochemical analytical means (GEO).

13. Installation according to one of the preceding claims, in which said container (2) of said fluid to be analysed is a bottom sampler.

14. Use of a mobile analytical installation (1) according to one of the preceding claims for analysing a fluid withdrawn from an underground formation of a site for the storage of gas, for the production of hydrocarbons, for geothermal heat or for the production of natural hydrogen.

15. Use according to Claim 14, in which the analysis is carried out of the saturation pressure, the volume of the gases present in the fluid to be withdrawn, and the composition of the gases present in said withdrawn fluid.

16. Use according to either of Claims 14 and 15, in which said mobile analytical installation (1) is moved on a site for withdrawal of said fluid to be analysed.

17. Use according to one of Claims 14 to 16, in which the following stages are carried out:
a) a container (2) of said fluid to be analysed is put into said mobile analytical installation (1);
b) said fluid to be analysed is transferred from said container (2) towards said plug-flow cells (3);
c) a PVT analysis is carried out starting from said fluid contained in a first plug-flow cell (3); and
d) at least one geothermal analysis (GEO) is carried out starting from said fluid contained in at least one second plug-flow cell (3).
